# FASCICULE DE BREVET EUROPEEN

(11) **EP 0 973 758 B1**
(45) Date de publication et mention de la délivrance du brevet: **30.01.2002**
(21) Numéro de dépôt: 98913851.6
(22) Date de dépôt: 06.03.1998
(51) Int. Cl.: C07D 301/02

(54) **PROCEDE DE FABRICATION D'UN EPOXYDE, NOTAMMENT DE GLYCIDOL, ET INSTALLATION DE MISE EN OEUVRE**
VERFAHREN ZUR HERSTELLUNG VON EINEM EPOXID, INSBESONDERE VON GLYCIDOL, UND AUSFÜHRUNGSVORRICHTUNG
METHOD FOR PRODUCING AN EPOXIDE, IN PARTICULAR OF GLYCIDOL, AND INSTALLATION FOR IMPLEMENTATION

(30) Priorité: 12.03.1997 FR 9703163
(43) Date de publication de la demande: 26.01.2000
(73) Titulaire: ORGANISATION NATIONALE INTERPROFESSIONNELLE DES OLEAGINEUX- ONIDOL, 75008 Paris (FR)
(72) Inventeur: YOO, Jeong-Woo, F-31500 Toulouse (FR); MOULOUNGUI, Zéphirin, F-31500 Toulouse (FR); GASSET, Antoine, F-31000 Toulouse (FR)
(74) Mandataire: Cabinet BARRE LAFORGUE & associés
(86) Numéro de dépôt international: FR9800451
(87) Numéro de publication internationale: WO9840371

(56) Documents cités:
- EP-A- 0 047 474
- EP-A- 0 180 387
- EP-A- 0 193 228
- US-A- 2 636 040
- US-A- 2 856 413
- US-A- 4 265 821
- US-A- 4 276 223
- US-A- 4 374 259
- US-A- 4 387 237
- US-A- 5 003 115

## Description

L'invention concerne un procédé de fabrication d'un époxyde de formule : 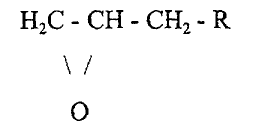
où R est un groupement hydroxyle OH pour le glycidol,
ou un radical alcoxyde O-R' avec R' = alkyle ou acyle pour les composés glycidyle,
ou un hydrogène H ou un groupement alkyle pour les oxydes d'alkylène.

L'invention s'applique en particulier pour la fabrication du glycidol : 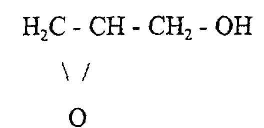 ou de composés glycidyles (dans lesquels l'hydrogène de l'hydroxyle est remplacé par un radical alkyle ou acyle).

Les époxydes ont de nombreuses applications comme adhésifs, revêtements, produits intermédiaires de fabrication de polyuréthanes .... Le glycidol et ses dérivés glycidyles sont une des familles importantes des époxydes et sont très utilisés dans les industries textile, plastique, pharmaceutique, cosmétique, photochimique, industrie des détergents, le bâtiment et les travaux publics en raison de leurs propriétés de stabilisateurs, de modificateurs de plastique, de surfactants, ignifuge... Depuis le début des années 1970, le glycidol est industriellement fabriqué par oxydation de l'alcool allylique au moyen de peroxyde d'hydrogène en présence d'un catalyseur à base d'oxyde de tungstène. Le défaut essentiel de ce procédé réside dans le grand nombre d'étapes nécessaires pour obtenir l'oxydation précitée, pour extraire le glycidol du milieu homogène aqueux (contenant le réactif de départ, et plusieurs produits secondaires : glycérol, acroléine, bétahydroxy-propionaldéhyde, éther allylique de glycérol, produits de décomposition du catalyseur) et pour purifier ensuite le glycidol obtenu en milieu aqueux. En outre, le catalyseur à base d'oxyde de tungstène est décomposé au cours de la réaction et sa consommation est un facteur d'augmentation du coût (DE-OS 2037703, US 3 625 981).

Il convient de noter que, avant ce procédé de fabrication à partir de l'alcool allylique, il avait été proposé d'autres procédés de fabrication du glycidol, soit à partir de glycérol et de carbonate d'éthylène, de propylène ou de butylène (brevet US 2 636 040), soit à partir de carbonate de glycérol (brevet US 2 856 413). De telles fabrications, si elles étaient transposables sur le plan industriel, seraient particulièrement intéressantes car les produits de départ peuvent être facilement obtenus à partir de matières végétales oléagineuses, offrant ainsi un débouché à ces matières. Toutefois, le procédé proposé dans le premier brevet (US 2 636 040) qui consiste à réaliser un complexe intermédiaire à partir du carbonate d'éthylène, propylène ou butylène et à décomposer celui-ci, présente l'inconvénient d'exiger une succession d'étapes à des conditions opératoires différentes, certaines à des pressions très basses ; de plus, le rendement effectif du procédé est relativement faible (63 %). Le procédé visé dans le second brevet (US 2 856 413) qui consiste à décomposer le carbonate de glycérol puis à synthétiser le glycidol en présence d'un sel métallique solubilisé dans le carbonate de glycérol, présente également l'inconvénient d'exiger des étapes successives à conditions opératoires différentes (décomposition à très basse pression, synthèse du glycidol, distillation) ; les mêmes commentaires que précédemment peuvent être formulés. De plus, les inventeurs de la présente invention ont effectué des essais dans les conditions de ce brevet et ont constaté que, au cours de la réaction, se développe un polymère solide très adhérent qui se dépose sur les parois du réacteur et peu à peu le colmate.

L'invention se propose de fournir un procédé de fabrication de glycidol ou d'un composé glycidyle, ou de façon plus générale d'époxyde de formule précédemment indiquée à partir d'un carbonate organique cyclique, en particulier carbonate de glycérol ou d'un dérivé de ce composé, pouvant être mis en oeuvre en une seule étape, notamment en continu, et susceptible d'opérer à des pressions supérieures aux pressions réduites exigées par les procédés ci-dessus décrits.

Un autre objectif est d'obtenir un rendement effectif élevé (le rendement effectif étant défini par le nombre de moles d'époxyde recueilli au nombre de moles de carbonate organique cyclique utilisé).

Un autre objectif est d'isoler directement l'époxyde, notamment le glycidol ou le composé glycidyle, et d'atteindre une pureté élevée de ce composé.

A cet effet le procédé visé par l'invention utilise comme composé de départ du carbonate organique cyclique à 5 chaînons de formule: 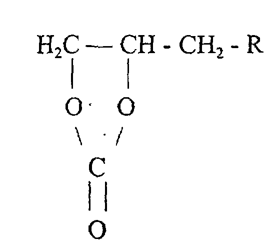 où
R = OH (pour le carbonate de glycérol)
R = H pour le carbonate de propylène,
R = alkyle pour les carbonates d'alkylène,
R = O - R' avec R' = alkyle ou acyle pour les autres carbonates organiques cycliques.

Le procédé visé est du type dans lequel on chauffe sous pression réduite le carbonate organique cyclique à une température au moins égale à 165° C de façon à réaliser une réaction de contraction du cycle carbonate :

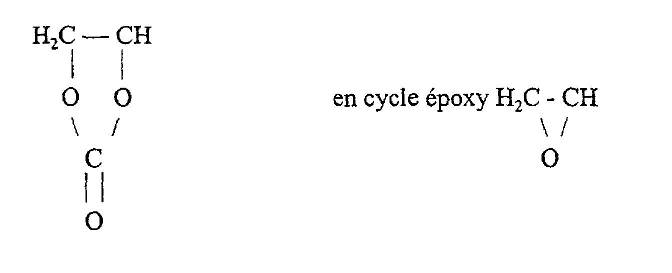

Le procédé de l'invention se caractérise en ce que la réaction est réalisée dans un système solide/liquide en présence d'un polyol et d'un catalyseur solide constitué par une zéolite de type A ou la γ-alumine.

Dans le procédé de l'invention, la réaction mise en oeuvre permet de former en une seule étape (donc avec une unicité des conditions opératoires) l'époxyde, en particulier glycidol ou dérivé glycidyle, qui se dégage sous forme de vapeur qu'il suffit d'extraire et de séparer du dioxyde de carbone formé (par simple condensation). On obtient alors directement un époxyde de bonne pureté (supérieure à 92 %). Cette réaction peut être facilement mise en oeuvre en continu par extraction continue de la phase gazeuse et séparation continue par condensation. Les expérimentations ont permis de constater, en particulier dans le cas de la fabrication du glycidol, que la réaction n'exigeait pas des pressions réduites très contraignantes et pouvait notamment être mise en oeuvre à des pressions comprises entre sensiblement 3.10³ et 10.10³ Pascals à des températures sensiblement comprises entre 170° C et 210° C ; en particulier une plage de pression entre 3,3.10³ et 6.10³ Pascals donne d'excellentes performances tout en conduisant à des coûts modérés de mise en oeuvre industrielle. Les essais ont montré que le rendement effectif en époxyde était de l'ordre de 75 % à 90 %.

Le procédé de l'invention se caractérise donc par la présence dans le milieu réactif :
. d'une part d'un polyol qui peut être avantageusement constitué par le glycérol ou un polyglycérol,
. d'autre part, d'un catalyseur solide spécifique : zéolite de type A, préférentiellement de forme H ou contenant un cation alcalin ou alcalino terreux, ou γ-alumine.

Les mécanismes qui conduisent à l'époxyde dans les conditions opératoires du procédé de l'invention sont nouveaux dans le type de réaction concerné. En premier lieu, le polyol remplit une première fonction de vecteur du carbonate organique évitant la décomposition de ce composé à la température du milieu et permettant sa diffusion et son accès vers les sites catalytiques de la zéolite ou de la γ-alumine ; le carbonate organique peut ainsi venir s'adsorber sur les sites catalytiques de la zéolite ou de la γ-alumine. Le carbonate organique une fois adsorbé sur ces sites catalytiques est apte à s'ouvrir compte tenu de la pression et de la température du milieu et le polyol remplit alors une deuxième fonction de donneur de protons permettant cette ouverture et la contraction du cycle carbonate en cycle époxy. L'époxyde ainsi formé est gazeux à la pression et à la température du milieu et diffuse hors du site catalytique. De plus, on n'observe pas de dépôt goudronneux en cours ou au terme du procédé et il semble que ce soit le polyol qui évite ces dépôts (troisième fonction du polyol) : il contribue ainsi à augmenter le rendement. Les études semblent montrer que les trois fonctions sus-indiquées du polyol sont remplies de façon optimale lorsque l'on dispose dans le milieu sensiblement entre 0,1 et 0,4 mole de polyol par mole de carbonate organique cyclique.

La zéolite utilisée est de préférence une poudre de granulométrie moyenne comprise entre 3 et 5 microns. On peut avantageusement disposer entre 3,5 et 10,5 g de zéolite par mole de carbonate organique cyclique afin que le milieu contienne un nombre de sites catalytiques non limitant pour la réaction.

Le procédé de l'invention peut être mis en oeuvre en "batch" ou en continu ou en semi-continu. La mise en oeuvre en continu ou semi-continu consiste à extraire en continu du milieu les vapeurs d'époxyde et le dioxyde de carbone formés ; cette mise en oeuvre sera préférée industriellement en raison des avantages auxquels elle conduit (rendement plus élevé, économie de catalyseur, réduction de la quantité de sous-produits, productivité élevée de l'installation).

Dans la mise en oeuvre semi-continue, du carbonate de glycérol est ajouté au cours de la réaction pour compenser les quantités consommées et maintenir sensiblement constante la concentration initiale de ce composé. Du polyol est également ajouté au cours de la réaction de façon à maintenir la proportion de polyol sensiblement comprise entre 0,1 et 0,4 mole de polyol par mole de carbonate de glycérol. Ces ajouts peuvent être effectués de sorte que le volume de la phase liquide du milieu réactif demeure constant afin d'obtenir une production continue sensiblement constante de glycidol. Dans cette mise en oeuvre semi-continue, on soutire de temps à autres par la partie basse du réacteur les autres constituants du milieu réactionnel.

La mise en oeuvre en continu comprend essentiellement les opérations suivantes : on prépare préalablement en continu un mélange de carbonate de glycérol, de glycérol et de catalyseur solide sous forme de poudre, on délivre en continu ce mélange dans un réacteur à film mince adapté pour centrifuger le mélange et former un film périphérique tournant constituant le milieu réactionnel, on extrait en continu en partie haute du réacteur les vapeurs de glycidol ou composé glycidyle et le dioxyde de carbone formés, et on extrait en continu en partie basse du réacteur les autres constituants du milieu.

Un tel procédé est intéressant sur le plan industriel en raison du caractère totalement continu du processus de fabrication et des économies d'énergie dues au déroulement de la réaction en film mince tournant.

L'invention s'étend à une installation pour la mise en oeuvre du procédé défini plus haut, caractérisée en ce qu'elle comprend en combinaison :
- des moyens d'alimentation en carbonate organique cyclique,
- des moyens d'alimentation en polyol,
- un réacteur fermé associé à des moyens de chauffage et à des moyens d'agitation et relié aux moyens d'alimentation précités,
- des moyens de prélèvement de la phase gazeuse du réacteur,
- un condenseur relié aux moyens de prélèvement en vue de condenser et séparer l'époxyde du CO₂,
- des moyens de récupération du glycidol liquide issu du condenseur,
- des moyens de pompage, reliés au condenseur et adaptés pour extraire le CO₂ de celui-ci et maintenir une pression réduite prédéterminée dans l'installation,
- des moyens de soutirage des constituants en partie basse du réacteur,
- les moyens d'alimentation, le réacteur, les moyens de prélèvement, le condenseur, les moyens de récupération et les moyens de soutirage formant un ensemble étanche relié aux moyens de pompage.

Le réacteur peut avantageusement être un réacteur à film mince équipé d'un mobile de centrifugation du milieu réactionnel. Il est à noter que ce type de réacteur à film mince est connu en soi pour réaliser des évaporations mais, à la connaissance des inventeurs, n'a jamais été utilisé pour réaliser une réaction, en particulier une réaction équilibrée. Ce type de réacteur favorisant l'évaporation sur toute l'interface de film mince contribue à un déplacement de l'équilibre de la réaction vers l'époxyde.

Les exemples qui suivent illustrent l'invention et les performances obtenues. Ces exemples ont été mis en oeuvre sur des installations telles que schématisées aux dessins ; sur ces dessins :
- la figure 1 est un schéma d'une installation à fonctionnement discontinu ("batch"), utilisée pour la mise en oeuvre des exemples 1 à 15,
- la figure 2 est un schéma d'une installation à fonctionnement semi-continu, utilisée pour la mise en oeuvre des exemples 16 à 20,
- la figure 3 est un schéma d'une installation à fonctionnement continu, utilisée pour la mise en oeuvre de l'exemple 21, et la figure 4 un schéma de détail du mobile de centrifugation du réacteur de cette installation.

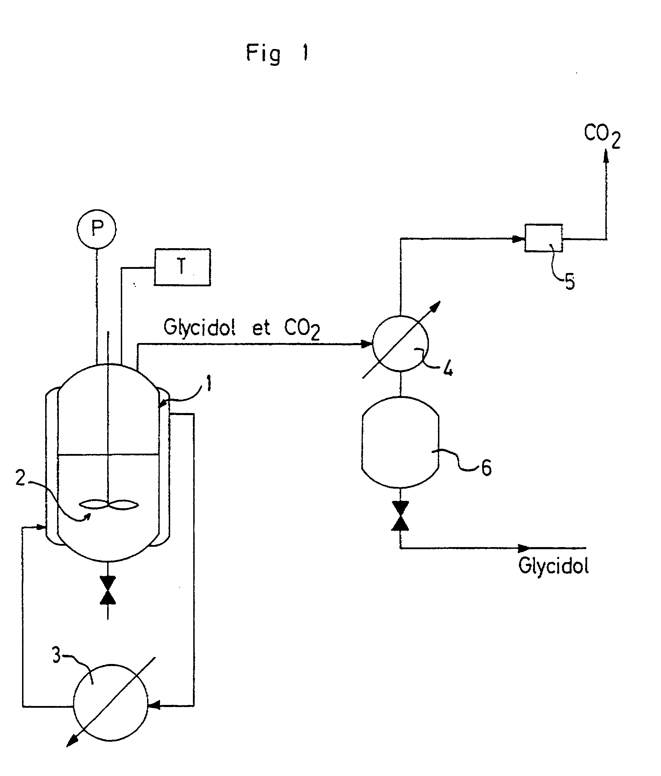

L'installation à fonctionnement discontinu, représentée à la figure 1, comprend un réacteur semi-fermé 1 dans lequel est disposé initialement un catalyseur sous forme de poudre solide et un mélange liquide de carbonate de glycérol et de glycérol. Ce réacteur est pourvu d'un agitateur mécanique symbolisé en 2 et de moyens de chauffage comprenant un liquide caloporteur circulant dans une double enveloppe et maintenu à la température appropriée par une résistance 3. Ce réacteur est relié par un conduit à un condenseur 4, lequel est associé, d'une part, à des moyens de pompage 5 permettant d'extraire la phase gazeuse et de maintenir dans l'installation et dans le réacteur 1 une pression réduite prédéterminée, d'autre part, à un ballon 6 de récupération du glycidol liquide formé.

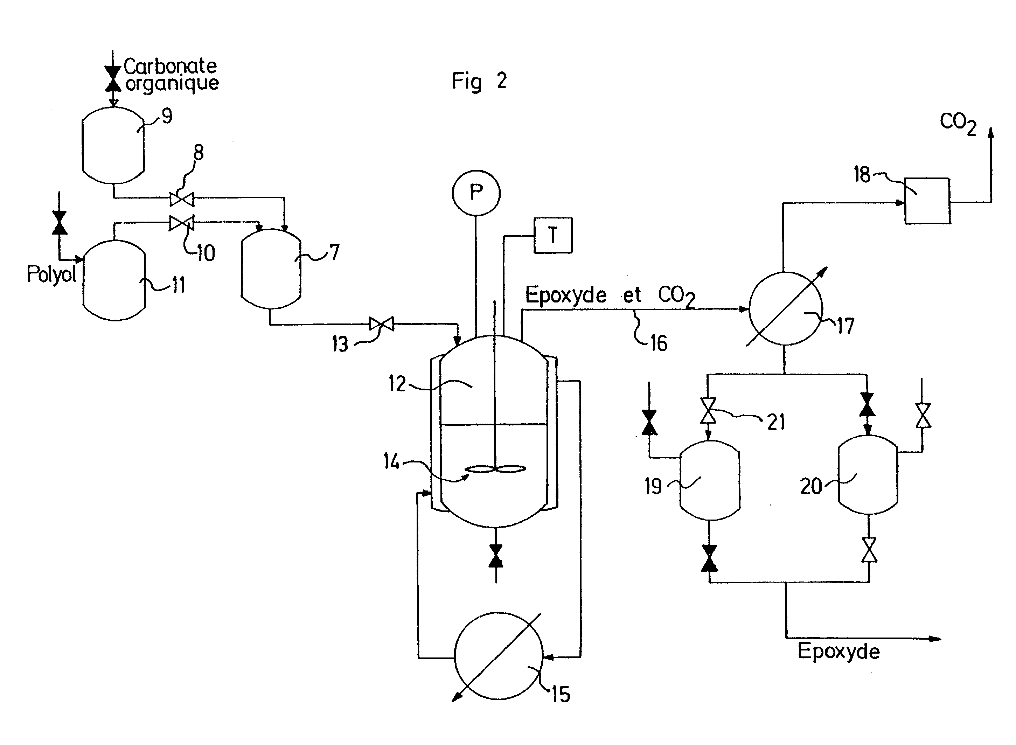

L'installation à fonctionnement semi-continu, représentée à la figure 2, comprend un mélangeur 7 relié d'une part par l'entremise de moyens de dosage 8 à un réservoir 9 de carbonate de glycérol, d'autre part, par l'entremise des moyens de dosage 10 à un réservoir 11 de glycérol. Le mélangeur 7 est relié à un réacteur 12 par l'entremise d'un doseur 13. Le réacteur 12 est doté de moyens d'agitation symbolisés en 14 et de moyens de chauffage symbolisés en 15 ; comme précédemment, le réacteur 12 est constitué par deux enveloppes entre lesquelles circule un fluide de chauffage permettant de réguler sa température. Un conduit 16 de prélèvement de la phase gazeuse du réacteur débouche dans un condenseur 17 contenant un réfrigérant, ce condenseur étant relié comme précédemment à des moyens de pompage 18 en vue de l'extraction du CO₂ et du maintien d'une pression réduite dans l'installation. Les moyens de récupération du glycidol liquide issu du condenseur comprennent, dans ce mode de réalisation, deux ballons 19 et 20 utilisés alternativement grâce à un jeu de vannes telles que 21. L'installation forme un ensemble étanche relié aux moyens de pompage 18 afin de permettre le maintien d'une pression réduite désirée dans ladite installation.

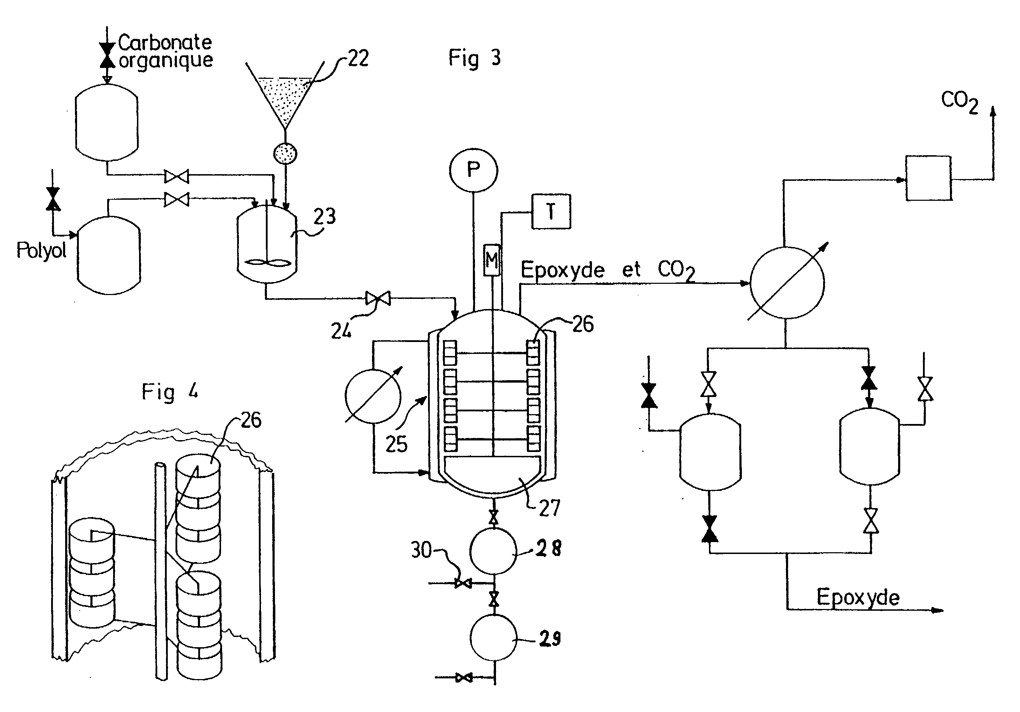

L'installation à fonctionnement continu représentée aux figures 3 et 4 est similaire à l'installation semi-continue de la figure 2 aux différences près suivantes :
Des moyens d'alimentation continue en catalyseur solide sous forme de poudre (trémie 22 et doseur) sont prévus pour délivrer un débit ajustable de poudre de catalyseur dans un mélangeur 23. Des moyens de dosage 24 alimentent ainsi un réacteur à film mince 25 en mélange réactionnel : carbonate organique cyclique, polyol, catalyseur en poudre ; ce mélange se présente sous la forme d'un liquide colloïdal facile à entraîner.
Le réacteur 25 est équipé d'un mobile de centrifugation composé de façon connue en soi d'un moteur d'entraînement M, de disques périphériques tournants tels que 26 (en l'exemple agencés en trois séries superposées, agencées à 120°) et d'une palette inférieure tournante 27. La vitesse de rotation du mobile de centrifugation est de 800 t/min en cours de fonctionnement ; le mobile engendre la formation d'un film mince (épaisseur inférieure au mm) contre la paroi cylindrique du réacteur.

A la base du réacteur, des moyens de soutirage permettent de prélever en continu les constituants du milieu autres que les constituants gazeux. Ces moyens de soutirage comprennent deux réservoirs 28 et 29 et des vannes telles que 30 qui permettent de vider tour à tour les réservoirs, tout en assurant un prélèvement continu par gravité à la base du réacteur et en maintenant le réacteur sous pression réduite.

Dans les trois types d'installations (discontinue, semi-continue ou continue), le réacteur est pourvu de capteurs de pression P et de température T.

### EXEMPLE 1 :

Dans le réacteur 1 semi-fermé de la figure 1, on mélange 35,4 g (0,3 mole) de carbonate de glycérol, 9,2 g (0,1 mole) de glycérol et 7,2 g de zéolite SA sous la forme Ca, à l'état de poudre de granulométrie moyenne égale à 4 µm. On porte le mélange réactionnel à 183° C et on abaisse la pression à 35 hectopascals sous agitation mécanique pendant 1 heure. Pendant la réaction, le glycidol condensé par le réfrigérant est récupéré dans le ballon 6. Le glycidol brut récupéré est analysé par chromatographie en phase gazeuse sur colonne capillaire "Carbowax" 20 M (12 m) avec le tétraéthylène glycol comme étalon interne. 25,6 g (0,217 mole) de carbonate de glycérol sont transformés en 16,1 g du glycidol soit un rendement molaire de 72 % par rapport au carbonate de glycérol.

### EXEMPLES 2-5 :

Une série d'essais de synthèse du glycidol est réalisée en utilisant le réacteur semi-fermé de la figure 1, dans lequel on mélange 35,4 g (0,3 mole) de carbonate de glycérol, 9,2 g (0,1 mole) de glycérol comme co-réactif et 7,2 g de catalyseur. La réaction est réalisée à une pression de 35 hectopascals. Dans ces exemples, on fait varier la nature du catalyseur. Les conditions opératoires et les résultats obtenus sont rassemblés dans le tableau 1.

**TABLEAU 1 :**

| Préparation du glycidol en présence de différents catalyseurs. | | | | |
|---|---|---|---|---|
| Numéro d'exemple | Catalyseur | Température (°C) | Durée (hr) | Rendement effectif en glycidol (%)* |
| 2 | Zéolite 4A, Na Poudre (3-5 µm) | 183-186 | 1 | 72 |
| 3 | Zéolite 3A, K Poudre (3-5 µm) | 183-188 | 1 | 72 |
| 4 | Zéobte A, H Poudre (3-5 µm) | 187-190 | 1 | 66 |
| 5 | Υ-Al₂O₃ | 178-186 | 4 | 44 |

| | | | | |
|---|---|---|---|---|
| * Rendement molaire en glycidol isolé par rapport au carbonate de glycérol. | | | | |

### EXEMPLES 6-7:

Une série d'essais de synthèse du glycidol est réalisée en utilisant le réacteur semi-fermé de la figure 1, dans lequel on mélange 35,4 g (o,3 mole) de carbonate de glycérol, 7,2 g de zéolite 5A comme catalyseur et 14 g de co-réactif. Les conditions opératoires et les résultats obtenus sont rassemblés dans le tableau 2.

**TABLEAU 2 :**

| Préparation du glycidol en présence de différents co-réactifs. | | | | |
|---|---|---|---|---|
| Numéro d'exemple | Co-réactif | Température (°C) | Durée (hr) | Rendement en glycidol (%)* |
| 6 | Polyéthyléne glycol 300 | 181-183 | 2 | 41 |
| 7 | Polyglycérol | 189-191 | 1 | 53 |

| | | | | |
|---|---|---|---|---|
| * Rendement molaire en glycidol isolé par rapport au carbonate de glycérol. | | | | |

### EXEMPLES 8-14 :

Une série d'essais de synthèse du glycidol est réalisée en utilisant le réacteur semi-fermé de la figure 1, dans lequel on mélange 35,4 g (0,3 mole) de carbonate de glycérol, le zéolite 5A et le glycérol. La réaction est réalisée sous vide partiel: Dans ces exemples, on fait varier la quantité de catalyseur et de glycérol, la température et la pression: Les conditions opératoires et les résultats obtenus sont rassemblés dans le tableau 3.

**TABLEAU 3 :**

| N° d'exemple | Concentration de glycérol (%)¹ | Quantité de zéolite 5A (g) | Température (°C) | Pression (hectopascals) | Durée (hr) | Rendement effectif en glycidol (%)² |
|---|---|---|---|---|---|---|
| 8 | 25 | 3,6 | 186-187 | 35 | 1 | 69 |
| 9 | 25 | 10,8 | 185-187 | 35 | 1 | 70 |
| 10 | 25 | 7,2 | 177-178 | 35 | 1 | 68 |
| 11 | 25 | 7,2 | 195-197 | 35 | 1 | 73 |
| 12 | 25 | 7,2 | 182-184 | 25 | 1 | 69 |
| 13 | 25 | 7,2 | 183-186 | 60 | 1 | 68 |
| 14 | 10 | 7,2 | 183-185 | 35 | 1 | 66 |

| | | | | | | |
|---|---|---|---|---|---|---|
| ¹ Concentration molaire de glycérol dans le mélange réactionnel | | | | | | |
| ² Rendement molaire en glycidol isolé par rapport au carbonate de glycérol. | | | | | | |

### EXEMPLE 15 :

Dans le réacteur de la figure 1, on mélangé 30,6 g (0,3 mole) de carbonate de propylène, 10 g (0,06 mole) de diglycérol et 5 g de zéolite 5A sous la forme Ca, à l'état de poudre de granulométrie moyenne égale à 4 µm. On porte le mélange réactionnel à 180° C et on abaisse la pression à 100 mbar sous agitation mécanique pendant 2 heures. Pendant la réaction, l'oxyde de propylène condensé par le réfrigérant est récupéré dans le ballon 7. Il est à noter que, à la sortie du réacteur 2, on veille à ce que le circuit soit exempt d'eau afin d'éviter une transformation de l'oxyde de propylène enpropanediol. L'oxyde de propylène brut récupéré est analysé par chromatographie en phase gazeuse. 9,6 g (0,16 mole) d'oxyde de propylène sont récupérés, soit un rendement molaire en oxyde de propylène de 53,3 % par rapport au carbonate de propylène.

### EXEMPLE 16 :

Dans le réacteur continu (500 ml) de la figure 2, on met initialement 2,4 g de zéolite 5A, 11,8 g (0,1 mole) de carbonate de glycérol et 2,7 (0,03 mole) de glycérol. On porte le mélange réactionnel à 183° C sous agitation mécanique pendant 5 minutes. Puis la réaction est réalisée sous 35 hectopascals. Pendant la réaction, on ajoute en continu un mélange de carbonate de glycérol et de glycérol à une vitesse d'alimentation de 1,1 ml/min. L'alimentation en réactifs et le soutirage du mélange gazeux de glycidol et CO₂ sont réalisés en continu pendant une heure et demie. Le glycidol condensé au niveau du réfrigérant est collecté dans les ballons 17 ou 18 de la figure 2. Le glycidol récupéré est analysé par chromatographie en phase gazeuse sur colonne capillaire "Carbowax" 20 M (12 m) avec le tétraéthylène glycol comme étalon interne. 59,6 g (0,51 mole) de carbonate, de glycérol sont transformés en 37,4 g du glycidol soit un rendement molaire de 84 % par rapport au carbonate de glycérol.

### EXEMPLES 17-18 :

Une série d'essais de synthèse du glycidol est réalisée en utilisant le réacteur parfaitement agité semi-continu de la figure 2. Le procédé, utilisé est celui de l'exemple 16. Les catalyseurs étudiés sont : le zéolite 4A et le zéolite 3A. Après l'analyse CPG, on peut observer les résultats rassemblés dans le tableau 4.

**TABLEAU 4 :**

| Préparation du glycidol dans le réacteur parfaitement agité semi-continu. Influence du catalyseur. | | | | |
|---|---|---|---|---|
| Numéro d'exemple | Catalyseur | Température (°C) | Durée (hr) | Rendement en glycidol (%)* |
| 16 | Zéolite 4A 2,4 g | 183-185 | 1,5 | 83 |
| 1.7 | Zéolite 3A 2,4 g | 183-185 | 1,5 | 83 |

| | | | | |
|---|---|---|---|---|
| * Rendement molaire en glycidol isolé par rapport au carbonate, de glycérol. | | | | |

### EXEMPLE 19 :

Un essai de synthèse du glycidol est réalisé en utilisant le réacteur parfaitement agité semi-continu de la figure 2. Le procédé utilisé est celui de l'exemple 16. La quantité de réactifs introduits au cours de la réaction est : 118 g (1 mole) de carbonate de glycérol et 31,3 g (0,34 mole) de glycérol. Après analyse CPG du glycidol isolé, on obtient 86 % de rendement molaire en glycidol isolé par rapport au carbonate de glycérol.

### EXEMPLE 20 :

Un essai de synthèse du glycidol est réalisé en utilisant le réacteur parfaitement agité semi-continu de la figure 2. Le procédé utilisé est celui de l'exemple 19. La quantité de zéolite 5A introduite est 4,9 g. Après l'analyse CPG du glycidol isqlé, on peut obtenir 85 % de rendement molaire en glycidol par rapport au carbonate de glycérol.

### EXEMPLE 21 :

On mélange 472 g (4 moles) de carbonate de glycérol, 73,6 g (0,8 mole) du glycérol et 28 g de zéolite A dans le mélangeur 23 de l'installation continue représentée à la figure 3. Le mélange réactionnel est distribué dans le réacteur avec une vitesse de 20 ml/minute par le doseur 24. La réaction est réalisée en film mince appliqué contre la surface intérieure cylindrique du réacteur pendant 30 minutes. La température de surface du réacteur est maintenue à 190-200° C par le fluide caloporteur (huile à 230° C). La réaction est réalisée sous 35 hectopascals (environ 35 mbar). L'alimentation des réactifs, le prélèvement du mélange gazeux de glycidol et CO₂ et le soutirage des constituants non gazeux au fond du réacteur sont réalisés en continu. Le glycidol condensé au niveau du réfrigérant est analysé par chromatographie en phase gazeuse (CPG) sur colonne capillaire "Carbowax 20M (12 m)" avec le tétraéthylène glycol comme étalon interne. Un rendement de 85 % avec une pureté de 92 %, est obtenu après l'analyse du CPG. Le produit recueilli contient 6 % de glycérol et 2 % de carbonate de glycérol qui sont entraînés par le glycidol et le CO₂.

## Revendications

1. Procédé de fabrication d'un époxyde de formule : 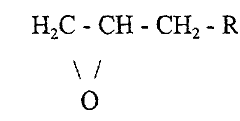 où R est le groupement hydroxyle OH pour le glycidol, ou un radical alcoxyde O-R' avec R' = alkyle ou acyle pour les composés glycidyle, ou un hydrogène H ou un groupement alkyle pour les oxydes d'alkylène,
dans lequel on chauffe sous pression réduite un carbonate organique cyclique à 5 chaînons : 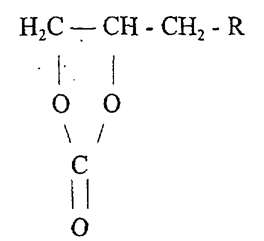 à une température au moins égale à 165° C de façon à réaliser une réaction de contraction du cycle carbonate en cycle époxy, **caractérisé en ce que** la réaction est réalisée dans un système solide/liquide en présence d'un polyol et d'un catalyseur solide constitué par une zéolite de type A ou de la γ-alumine.

2. Procédé de fabrication selon la revendication 1, dans lequel on utilise comme polyol le glycérol ou un polyglycérol.

3. Procédé de fabrication selon l'une des revendications 1 ou 2, **caractérisé en ce qu'**on utilise comme catalyseur solide une zéolite de forme H ou contenant un cation alcalin ou alcalino-terreux.

4. Procédé de fabrication selon la revendication 3, dans lequel la zéolite est mélangée au milieu sous la forme d'une poudre de granulométrie moyenne comprise entre 3 et 5 microns.

5. Procédé de fabrication selon l'une des revendications 3 ou 4, **caractérisé en ce qu'**on dispose dans le milieu sensiblement entre 3,5 et 10,5 g de zéolite par mole de carbonate organique cyclique.

6. Procédé de fabrication selon l'une des revendications 1, 2, 3, 4 ou 5, **caractérisé en ce qu'**on dispose dans le milieu sensiblement entre 0,1 et 0,4 mole de polyol par mole de carbonate organique cyclique.

7. Procédé selon l'une des revendications 1, 2, 3, 4, 5 ou 6 pour fabriquer du glycidol ou un composé glycidyle, dans lequel on utilise du carbonate de glycérol ou un dérivé comme carbonate organique cyclique et du glycérol comme polyol.

8. Procédé de fabrication selon la revendication 7, dans lequel on chauffe le milieu à une température sensiblement comprise entre 170° C et 210° C et on ajuste la pression à une valeur sensiblement comprise entre 3.10³ et 10.10³ Pascals, en particulier entre 3,3.10³ et 6.10³ Pascals.

9. Procédé de fabrication selon l'une des revendications 7 ou 8, **caractérisé en ce qu'**on extrait en continu du milieu les vapeurs de glycidol ou composé glycidyle et le dioxyde de carbone formés, et on sépare par condensation le glycidol ou composé glycidyle.

10. Procédé selon la revendication 9, pour la fabrication en semi-continu de glycidol ou composé glycidyle, **caractérisé en ce que**, au cours de la réaction, on ajoute dans le milieu du carbonate de glycérol de façon à maintenir sensiblement sa concentration initiale, et du polyol de façon à maintenir la proportion de polyol sensiblement comprise entre 0,1 et 0,4 mole de polyol par mole de carbonate de glycérol.

11. Procédé selon la revendication 9 pour la fabrication en continu de glycidol ou composé glycidyle, **caractérisé en ce qu'**on prépare préalablement en continu un mélange de carbonate de glycérol, de glycérol et de catalyseur solide sous la forme de poudre, on délivre en continu ce mélange dans un réacteur à film mince adapté pour centrifuger le mélange et former un film périphérique tournant constituant le milieu réactionnel, on extrait en continu en partie haute du réacteur les vapeurs de glycidol ou composé glycidyle et le dioxyde de carbone formés, et on extrait en continu en partie basse du réacteur les autres constituants du milieu.

12. Installation de fabrication d'un époxyde en vue de la mise en oeuvre du procédé conforme à l'une des revendications 1 à 10, **caractérisée en ce qu'**elle comprend en combinaison :
- des moyens (9, 8, 7, 13) d'alimentation en carbonate organique cyclique,
- des moyens (11, 10, 7, 13) d'alimentation en polyol,
- un réacteur (12) associé à des moyens de chauffage (15) et à des moyens d'agitation (14) et relié aux moyens d'alimentation précités,
- des moyens (16) de prélèvement de la phase gazeuse du réacteur,
- un condenseur (17) relié aux moyens de prélèvement en vue de condenser et séparer l'époxyde du CO₂,
- des moyens (19, 20, 21) de récupération de l'époxyde liquide issu du condenseur,
- des moyens de pompage (18), reliés au condenseur en vue de l'extraction du CO₂ et du maintien d'une pression réduite prédéterminée dans l'installation,
- des moyens de soutirage des constituants en partie basse du réacteur,
- les moyens d'alimentation, le réacteur, les moyens de prélèvement, le condenseur, les moyens de récupération et les moyens de soutirage formant un ensemble étanche relié aux moyens de pompage.

13. Installation selon la revendication 12 pour la fabrication en continu d'un époxyde, **caractérisée en ce que** le réacteur est un réacteur à film mince (25) équipé d'un mobile de centrifugation du milieu réactionnel (26, 27).

## Patentansprüche

1. Verfahren zur Herstellung eines Expoxidharzes der. Formel 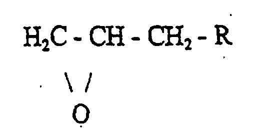 wobei R die Hydroxylgruppe OH für das Glycidol oder ein Alkoxidradikal O-R' ist, wobei R'= Alkyl oder Acyl für die Glycidylverbindungen oder ein Wasserstoffatom H oder eine Alkylgruppe für die Alkylenoxide ist,
bei dem ein zyklisches organisches Carbonat mit 5 Ketten: 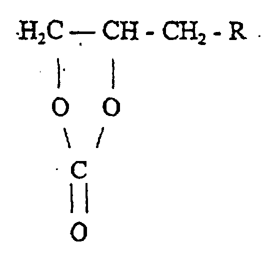 unter verringertem Druck auf eine Temperatur von wenigstens 165°C erhitzt wird, um eine Kontraktionsreaktion des Carbonatrings in einen Epoxidharzring zu erzielen, **dadurch gekennzeichnet, dass** die Reaktion in einem festen/flüssigen System in Anwesenheit eine Polyols und eines festen Katalysators erfolgt, der aus einem Zeolith des Typs A oder aus γ-Aluminiumoxid besteht.

2. Herstellungsverfahren nach Anspruch 1, bei dem Glycerol oder ein Polyglycerol als Polyol verwendet wird.

3. Herstellungsverfahren nach Anspruch 1 oder 2, **dadurch gekennzeichnet, dass** als fester Katalysator ein Zeolith der H-Form oder ein alkalihaltiges oder ein erdalkalisches Kation verwendet wird.

4. Herstellungsverfahren nach Anspruch 3, bei dem das Zeolith mit dem Medium in der Form eines Pulvers mit einer mittleren Körnchengröße zwischen 3 und 5 Mikron gemischt wird.

5. Herstellungsverfahren nach Anspruch 3 oder 4, **dadurch gekennzeichnet, dass** dem Medium im Wesentlichen zwischen 3,5 und 10,5 g Zeolith pro Mol zyklisches organisches Carbonat zugegeben werden.

6. Herstellungsverfahren nach einem der Ansprüche 1, 2, 3, 4 oder 5, **dadurch gekennzeichnet, dass** dem Medium im Wesentlichen zwischen 0,1 und 0,4 Mol Polyol pro Mol zyklisches organisches Carbonat zugegeben werden.

7. Verfahren nach einem der Ansprüche 1, 2, 3, 4, 5 oder 6 zur Herstellung des Glycidols oder einer Glycidylverbindung, bei dem Glycerolcarbonat oder ein Derivat als zyklisches organisches Carbonat und Glycerol als Polyol verwendet werden.

8. Herstellungsverfahren nach Anspruch 7, bei dem das Medium auf eine Temperatur im Wesentlichen zwischen 170°C und 210°C erhitzt und der Druck auf einen Wert im Wesentlichen zwischen 3 x 10³ und 10 x 10³ Pascal, insbesondere zwischen 3,3 x 10³ und 6 x 10³ Pascal eingestellt wird.

9. Herstellungsverfahren nach Anspruch 7 oder 8, **dadurch gekennzeichnet, dass** aus dem Medium kontinuierlich die gebildeten Dämpfe von Glycidol oder der Glycidylverbindung und des Kohlendioxid extrahiert werden und das Glycidol oder die Glycidylverbindung durch Kondensation abgeschieden wird.

10. Verfahren nach Anspruch 9 zur halbkontinuierlichen Herstellung von Glycidol oder einer Glycidylverbindung, **dadurch gekennzeichnet, dass** im Verlaufe der Reaktion dem Medium Glycerolcarbonat zugegeben wird, so dass seine Anfangskonzentration im Wesentlichen erhalten bleibt, und Polyol, so dass der Polyolanteil im Wesentlichen zwischen 0,1 und 0,4 Mol Polyol pro Mol Glycerolcarbonat bleibt.

11. Verfahren nach Anspruch 9 zur kontinuierlichen Herstellung von Glycidol oder einer Glycidylverbindung, **dadurch gekennzeichnet, dass** zuvor kontinuierlich ein Gemisch aus Glycerolcarbonat, Glycerol und festem Katalysator in Pulverform hergestellt wird, dieses Gemisch kontinuierlich in einen Dünnfilmreaktor gegeben wird, um das Gemisch zu zentrifugieren und einen rotierenden peripheren Film zu bilden, der das Reaktionsmedium darstellt, in einem oberen Teil des Reaktors kontinuierlich die gebildeten Glycidol- oder Glycidylverbindungs- und Kohlendioxiddämpfe extrahiert und in einem unteren Teil des Reaktors kontinuierlich die übrigen Bestandteile des Mediums extrahiert werden.

12. Anlage zur Herstellung eines Epoxidharzes zur Durchführung des Verfahrens nach den Ansprüchen 1 bis 10, **dadurch gekennzeichnet, dass** sie in Kombination folgendes umfasst:
- Mittel (9, 8, 7, 13) zum Zuführen von zyklischem organischem Carbonat,
- Mittel (11, 10, 7, 13) zum Zuführen von Polyol,
- einen Reaktor (12) in Verbindung mit Heizmitteln (15) und Rührmitteln (14) und verbunden mit den oben genannten Zuführungsmitteln,
- Mittel (16) zum Entnehmen der Gasphase des Reaktors,
- einen Kondensator (17) in Verbindung mit den Entnahmemitteln zum Kondensieren und Abscheiden des Epoxidharzes vom CO₂,
- Mittel (19, 20, 21) zur Rückgewinnung des aus dem Kondensator austretenden flüssigen Epoxidharzes,
- Pumpmittel (18) in Verbindung mit dem Kondensator, um das CO₂ zu extrahieren und auf einem in der Anlage vorbestimmten reduzierten Druck zu halten,
- Mittel zum Abziehen von Bestandteilen im unteren Teil des Reaktors,
- wobei die Zuführungsmittel, der Reaktor, die Entnahmemittel, der Kondensator, die Rückgewinnungsmittel und die Abzugsmittel eine dichte Einheit in Verbindung mit dem Pumpmittel bilden.

13. Anlage nach Anspruch 12 für die kontinuierliche Herstellung eines Epoxidharzes, **dadurch gekennzeichnet, dass** der Reaktor ein Dünnfilmreaktor (25) ist, der mit einer Vorrichtung zum Zentrifugieren des Reaktionsmediums (26, 27) ausgestattet ist.

## Claims

1. A method for preparing an epoxide of the formula : 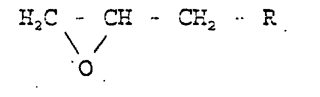 where R is a hydroxyl OH group for glycidol, or an alkoxide radical O-R' with R' = alkyl or acyl for glycidyl compounds, or a hydrogen H or an alkyl group for alkylene oxides,
wherein a cyclic organic carbonate with 5 links : 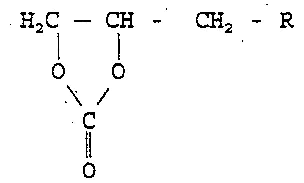 is heated under reduced pressure to a temperature at least equal to 165°C so as to perform a contraction reaction of the carbonate ring into an epoxy ring, wherein the reaction is carried out in a solid/liquid system in the presence of a polyol and a solid catalyst consisting of a type A zeolite or γ-alumina.

2. The preparative method as claimed in claim 1, wherein glycerol or a polyglycerol is used as the polyol.

3. The preparative method as claimed in one of claims 1 or 2, **characterized in that** a zeolite in the H form or containing an alkali metal or alkaline earth cation is used as the solid catalyst.

4. The preparative method as claimed in claim 3, wherein the zeolite is mixed into the medium in the form of powder with a mean particle size of between 3 and 5 microns.

5. The preparative method as claimed in one of claims 3 or 4, **characterized in that** use is made of between 3.5 and 10.5 g of zeolite per mole of cyclic organic carbonate.

6. The preparative method as claimed in one of claims 1, 2, 3, 4 or 5, **characterized in that** use is made in the medium of substantially between 0.1 and 0.4 mole of polyol per mole of cyclic organic carbonate.

7. The method as claimed in one of claims 1, 2, 3, 4; 5 or 6, for preparing glycidol or a glycidyl compound, wherein glycerol carbonate or a glycidyl compound is used as the cyclic organic carbonate and glycerol is used as the polyol.

8. The preparative method as claimed in claim 7, wherein the medium is heated to a temperature substantially between 170°C and 210°C and the pressure is adjusted to a value substantially between 3 x 10³ and 10 × 10³ Pascals, in particular between 3.3 x 10³ and 6 × 10³ Pascals.

9. The preparative method as claimed in one of claims 7 or 8, **characterized in that** the vapors formed of glycidol or glycidyl compound and the carbon dioxide are extracted continuously from the medium and the glycidol or glycidyl compound is separated by condensation.

10. The method as claimed in claim 9, for semi-continuously preparing glycidol or a glycidyl compound, **characterized in that**, during the reaction, glycerol carbonate is added to the reaction medium so as to keep substantially its initial concentration, and polyol is added so as to keep the proportion of polyol substantially between 0.1 and 0.4 mole of polyol per mole of glycerol carbonate.

11. The method as claimed in claim 9 for continuously preparing glycidol or a glycidyl compound, **characterized in that** a mixture of glycerol carbonate, glycerol and solid catalyst in the form of powder are previously prepared continuously and this mixture is continuously delivered into a thin-film reactor adapted to centrifuge the mixture and to form a revolving peripheral film constituting the reaction medium, vapours of glycidol or a glycidyl compound and the carbon dioxide formed are extracted continuously from the upper part of the reactor, and the other constituents of the medium are extracted continuously from the lower part of the reactor.

12. An installation for preparing an epoxide with a view to implementing the method as claimed in one of claims 1 to 10, **characterized in that** it comprises, in combination :
- means (9, 8, 7, 13) for supplying cyclic organic carbonate,
- means (11, 10, 7, 13) for supplying polyol.
- a reactor (12) associated with means of heating (15) and means of stirring (14) and connected to the aforementioned means of supply,
- means (16) for extracting the gaseous phase from the reactor,
- a condenser (17) connected to the extraction means with a view to condensing and separating epoxide from CO₂,
- means (19, 20, 21) for recovering the liquid epoxide issuing from the condenser,
- means of pumping (18), connected to the condenser with a view to extracting CO₂ and maintaining a predetermined reduced pressure in. the installation,
- means for drawing off the constituents from the lower part of the reactor,
- the means of supply, the reactor, the means of extraction, the condenser, the means of recovery and the draw-off means forming a sealed assembly connected to the means of pumping.

13. The installation as claimed in claim 12 for continuously preparing an epoxide, **characterized in that** the reactor is a thin-film reactor (25) equipped with a centrifuge for centrifuging the reaction medium (26, 27).
